# EUROPEAN PATENT SPECIFICATION

(11) **EP 4 051 360 B1**
(45) Date of publication and mention of the grant of the patent: **01.11.2023**
(21) Application number: 20811439.7
(22) Date of filing: 26.10.2020
(51) Int. Cl.: A61M 25/06, A61M 25/09, A61M 39/02, A61M 39/06, A61M 39/24

(54) **BIOMEDICAL DEVICE FOR ARTERIAL ACCESS**
BIOMEDIZINISCHE VORRICHTUNG FÜR ARTERIELLEN ZUGRIFF
DISPOSITIF BIOMÉDICAL POUR ACCÈS ARTÉRIEL

(30) Priority: 28.10.2019 IT 201900019842
(43) Date of publication of application: 07.09.2022
(73) Proprietor: Medi.Pro S.r.l., 73100 Lecce (IT)
(72) Inventor: PAPADIA, Gabriele, 73100 Lecce (IT)
(74) Representative: Bruni, Giovanni
(86) International application number: PCT/IB2020/060032
(87) International publication number: WO 2021/084403

(56) References cited:
- EP-A1- 2 569 046
- US-A1- 2014 214 005
- US-A1- 2015 051 584

## Description

### TECHNICAL FIELD

Object of the present invention is a biomedical device for arterial access and in particular a device for realizing arterial catheterization.

### PRIOR ART

Arteries are blood vessels for carrying blood from heart to all tissues and organs of the human body (centrifugal direction). With the exception of the pulmonary arteries, that carry blood charged with carbon dioxide and waste products, all the arteries carry oxygenated blood. The arterial system is the high-pressure part of the circulatory system and is made up of aorta artery, systemic arteries, pulmonary arteries. The arteries and arterial system task is to carry blood from heart to all the tissues and organs of the human body.

For arterial catheterization is intended the insertion of a cannula or catheter inside a palpable artery (with the exception of the carotid artery). Arterial catheterization allows monitoring of arterial pressure (systolic, average and diastolic) and quick blood sampling for arterial-blood gas test. This procedure is performed in patients admitted to intensive, sub-intensive care units and patients in operating room.

The arterial catheter is usually made up of biocompatible material, generally Teflon or polyurethane. This last one seems less associated with infective complications than catheters in polyvinyl chloride or polyethylene. The inner diameter is expressed in Gauge, for example in radial artery it is used a 20-22 Gauge catheter equivalent to 0.812-0.644 millimeters in diameter. The catheter size varies depending on the patient age (adult or child) and on the selected insertion site.

At the state of the art, it is also obviously known the peripheral and central venous catheterization. In particular, the central venous catheter (CVC), also called device for central venous access, is a medical device that allows to have access to greater caliber venous blood vessels. In comparison with the peripheral venous catheter the CVC guarantees a stable and safe access, through which it is possible to administer great volumes of solutions or drugs that require a high flow or solutions with too high osmolarity for peripheral administration. CVC is often made up of polyurethane but also silicone, it is biologically compatible, and it can have different independent lumens (one to five lumens).

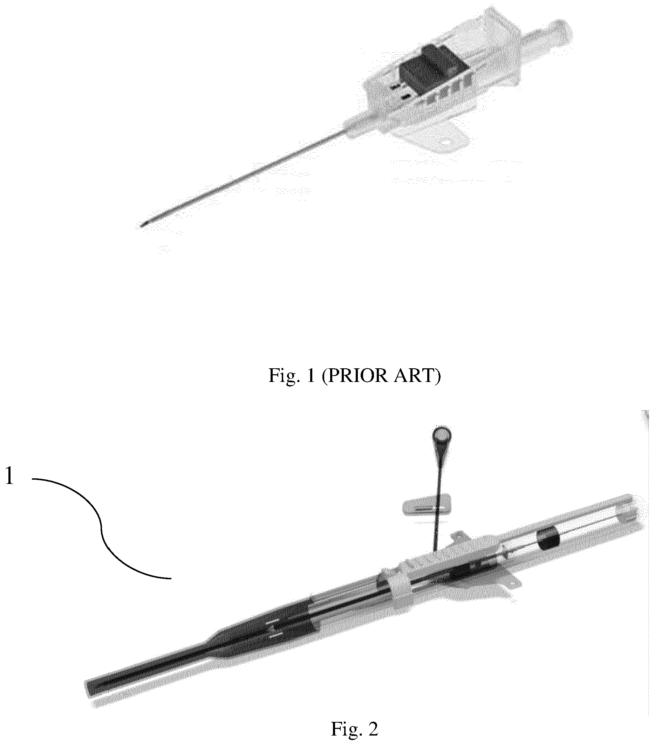

For example, with reference to fig. 1, a catheter for hemodialysis is a thin biocompatible tube realized in flexible material. The catheter is introduced in a "target" vein of a patient. The catheter is generally made up of a lumen (single or forked), a hub, an extension, a Luer, a clamp and sometimes a cuff. Moreover, the catheter can comprise also side holes, a strain relief, suture wings and one or more ID rings. All the components are generally made of polymeric material. Dialysis catheters are available in "single", "double" and "triple lumen" configurations.

According to the known Seldinger technique, in order to obtain safe access to blood vessels it is required to carry out a cutaneous puncture with a needle, to insert a guide in a blood vessel, to withdraw the needle from the blood vessel, to introduce the catheter inside the vessel after suitable dilatation of the vessel itself and to remove finally the guide previously inserted.

In this technical field, it is also known the Patent US2016106971A1, in which it is described a peripheral intravenous catheter with an internal bi-valvular secure system with the aim of avoiding backflow spilling of blood or any other biological substance during the insertion of a catheter. It is also known the Patent CN105326487A, which instead relates to a system with indwelling needle with a hemostasis valve provided with an external catheter, an inner needle in steel, a negative pressure cavity for blood return and a cap. The inner arterial needle provided with the hemostasis valve and the cavity for negative pressure backflow blood, which can be substituted, have the aim of avoiding the phenomenon of strong hemorrhages through the hemostasis valve, to reduce the possibility that the blood is polluted or to come into contact with it.

In other cases, there are some examples of applications relating to safety systems for needle clamping. Patents WO9908742A1, WO2015161294, WO2010127846 are among these ones.

Earlier documents, like US20150051584A1, EP2569046, US20140214005A1, are also known. In particular, in the United States Patent US20150051584A1 it is described a biomedical device for realizing an arterial catheterization comprising a catheter assembly, an outer sheath assembly, a Seldinger assembly, a cannula-needle integral to the Seldinger assembly, valve to avoid blood backflow and safety means for needle clamping, means for needle protection, said catheter assembly further comprising a thin tube, a nearly cylindrically shaped end provided with a couple of side wings, an internal cavity obtained in said end, wherein the outer sheath assembly is configured to contain therein both said catheter assembly and said Seldinger assembly when the device is assembled before its use and the valve means and the safety means are located inside the outer sheath assembly. The device according to Patent EP2569046 is not provided with a nearly cylindrically shaped end provided with a couple of side wings, so that it is not formed a cavity inside which valve means and safety means can be inserted for needle clamping. The United States Patent US20140214005A1 describes instead valve means arranged inside a cavity of the end of a catheter, free of safety means for needle clamping inside said cavity.

Contrary to venous access, arterial access often causes blood loss due to the high pressure in the arteries. Sometimes blood loss is very dangerous for nurses and doctors since, due to the pressure, blood spatter can hit them accidentally. Currently, the only possibility to reduce blood loss and spatter is linked to the operator skills who, manually, tries to reduce blood loss by applying a pressure with the fingers of one of both hands, while with the other one performs the access. Therefore, some problems known at the state of the art and, linked to the use of such devices, like for example the undesired blood loss during their usage or the accidental puncture of the operator during the catheterization procedure, remain unsolved.

### AIM OF THE INVENTION

Therefore, aim of the present invention is to solve the described disadvantages by using a valved system that blocks the needle to avoid accidental punctures of the operator.

Another aim of the invention is to avoid blood loss during arterial catheterization procedures by means of an additional system of valves that blocks blood backflow.

The object of the present invention reaches the aims of the invention since it is a biomedical device for arterial access with the technical specifications as expressed in the attached claims.

### DESCRIPTION OF THE FIGURES

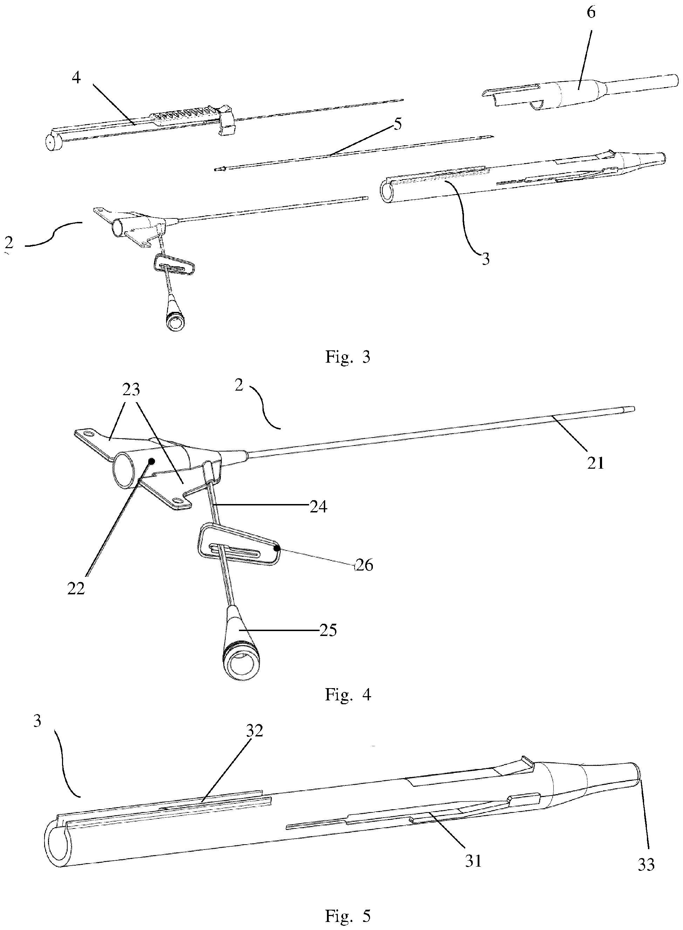

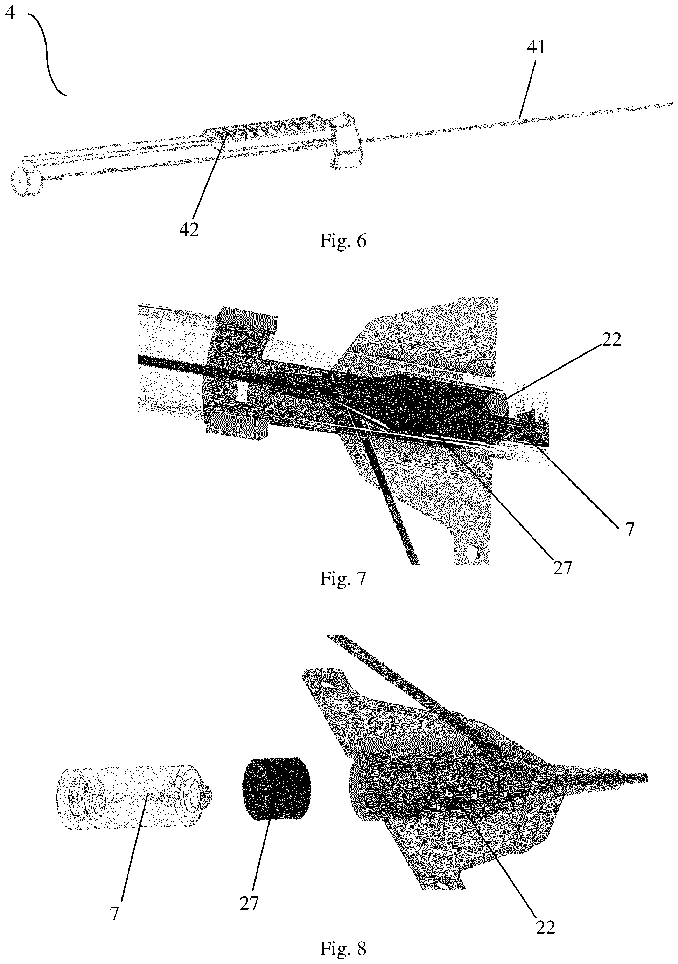

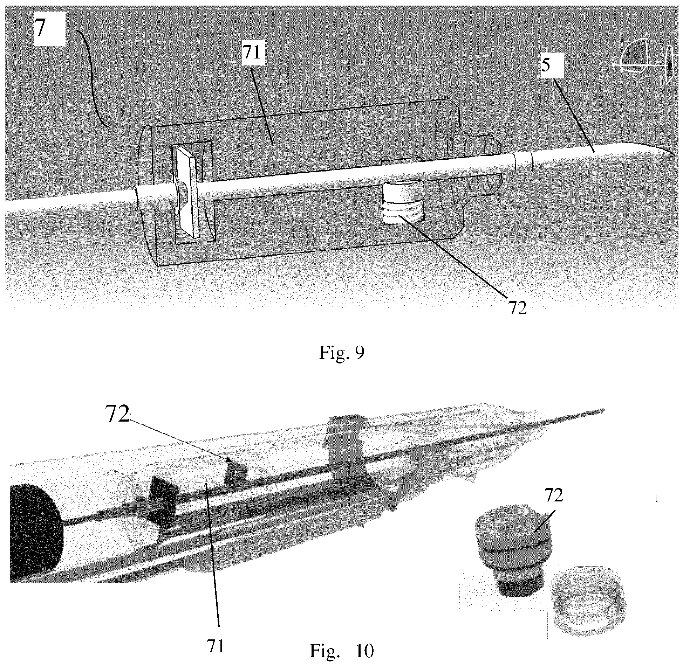

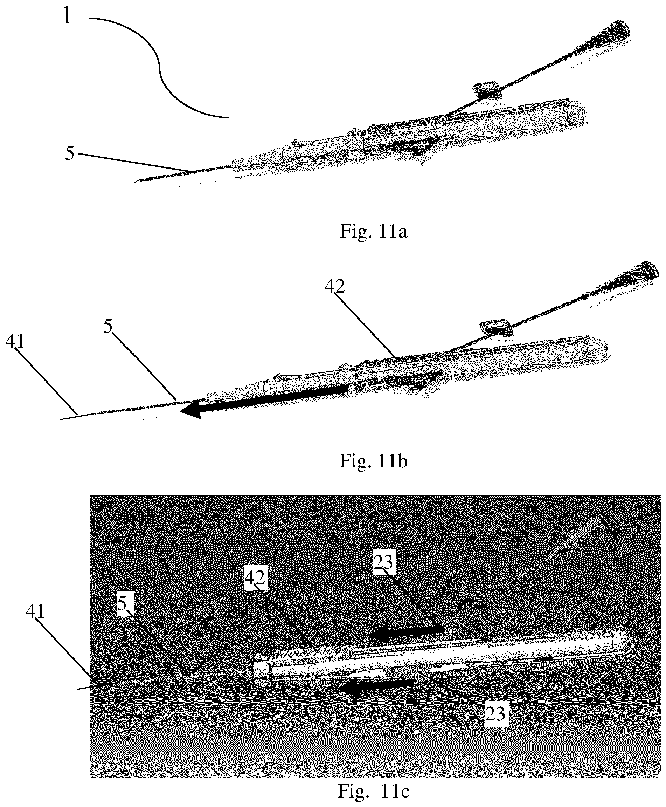

Figure 1 shows a peripheral arterial catheter known at the state of the art, figure 2 shows a panoramic view of the assembly of the device for arterial access which is an object of the present invention, figure 3 shows an exploded view of the components of the device for arterial access, figures 4 to 6 show a detailed view of catheter assembly (fig. 4), outer sheath assembly (fig. 5), Seldinger assembly (fig. 6) respectively, figures 7 and 8 show detailed views of valved systems the device for arterial access is provided with, figures 9 and 10 show detailed views of a system for device needle protection, figures 11a to 11f show a sequence of use of the device for arterial access.

### DETAILED DESCRIPTION

With reference to mentioned above figures, a preferred embodiment of the object of the present invention is described in detail as a way of not limiting example.

As yet stated, the device for arterial access, object of the present invention is a device comprising a double valvular system to compensate the arterial pressure and a needle clamping system. With reference to figures 2 and 3, the device (1) for arterial access is made up of assembly of the following main components: a catheter assembly (2), an outer sheath assembly (3) and a Seldinger assembly (4). The device 1 comprises also a cannula-needle (5) through which it is possible to perforate an artery, said cannula-needle (5) being substantially integral to the Seldinger assembly (4), as it will be described better below. A suitably shaped cap (6) is provided for cannula-needle protection. The outer sheath assembly (3) is configured to contain therein both the catheter assembly (2) and the Seldinger assembly (4) when the device is assembled as shown in fig. 2.

With reference to figures 4 to 10, the just mentioned main components of the device (1) will be described in detail. The catheter assembly (2) is composed of a thin tube (21), to be inserted inside the artery of the patient, a nearly cylindrically shaped end (22) provided with a couple of side wings (23) and an extension (24) of the catheter. Said catheter assembly (2) is configured to be assembled inside the outer sheath assembly (3) so that the thin tube (21) comes out from an open end of the outer sheath assembly (3) and so that said couple of wings (23) come out from a couple of side openings (31), opposite to each other positioned on the outer sheath assembly (3). Moreover, said end (22) of the catheter assembly (2) is provided with a cylindrical seat suitable to contain therein a first valve (27) as well as safety means (7), housed in the outer sheath assembly (3) and having the aim of avoiding the blood backflow and the needle tip clamping, respectively. The extension (24) of the catheter, instead, consists of a catheter shunt that allows to carry out operations such as, for example, the measurement of arterial pressure and blood sampling, by screwing a proper secondary device to an end (25) provided with a universal connection. Preferably, said connection is of "luer lock" type, or it is provided with a thread that allows to insert any tool provided with the same type of the connection. On this shunt of the catheter, it is also possible to use clamps (26) to interrupt the blood flow momentarily. The outer sheath assembly (3), as stated above, is configured to contain therein the catheter assembly (2), the Seldinger assembly (4) and the cannula-needle (5) when the device (1) is assembled. During the use of the device (1) for arterial access, said assembly (3) is also configured to be divided in two parts, subjected to the actions of both the catheter assembly (2) and the Seldinger assembly (4). As it is shown in figure 5, the outer sheath assembly (3) is provided with a couple of side openings (31), from which the wings (23) of the catheter assembly (2) come out. Moreover, on the upper part it is provided with a longitudinally developed opening (32) through which it is possible to introduce the Seldinger assembly (4) and in particular the sliding actuation means (42) of the Seldinger assembly (4). The thin tube (21) comes out from the open end (33) of the assembly (3), while the Seldinger assembly (4) is inserted from the other opposite, open end (34).

The Seldinger group (4) is made up of a sliding actuation means (42) to be introduced in the opening (32) of the outer sheath assembly (3) and of a guidewire (41) to be injected in the artery of the patient through the cannula-needle (5).

For easiness of representation, the safety and clamping means (7) for the needle are represented more in detail in figures 8 to 10. Said means (7) have the aim of avoiding undesired accidental punctures or contaminations for operators. Said clamping means (7), when the device is assembled, are located inside the outer sheath assembly (3) close to the valve (27) located in the cylindrical seat (22) of the catheter assembly (2). More in detail, and with reference to figures 9 and 10, said means are located inside a convex seat (71) and are made up of a spring bulkhead (72) configured to be closed when it is crossed by the needle tip (5). In such way, as it will be described better below, once the needle (5) is removed from the artery leaving the catheter thereinside, the spring bulkhead (72) closes the access for the tip of the needle (5) thus making it impossible to come out.

Therefore, with the device (1) assembled in all its parts, as shown in figure 2, it results that: catheter assembly (2) and Seldinger assembly (4) are assembled inside the outer sheath assembly (3) together with the cannula-needle (5) which can be considered integral to the Seldinger assembly (4). The thin tube (21) of the catheter assembly (2) contains thereinside the cannula-needle (5) and the guidewire (41) of the Seldinger assembly (4), respectively.

As a way of pure and not limiting example, a preferred embodiment of the assembly of the device (1) can be realized with the following geometrical features:
- needle length: 153 mm
- needle diameter: 0.80 = 21G
- catheter thin tube length: 100 mm
- catheter inner diameter: 1.00 mm
- catheter outer diameter: 1.35 mm = 18G
- outer diameter of the container shaft: 10 mm
- Seldinger stroke: 30 mm
- total device length (with Seldinger inside the device): 190 mm

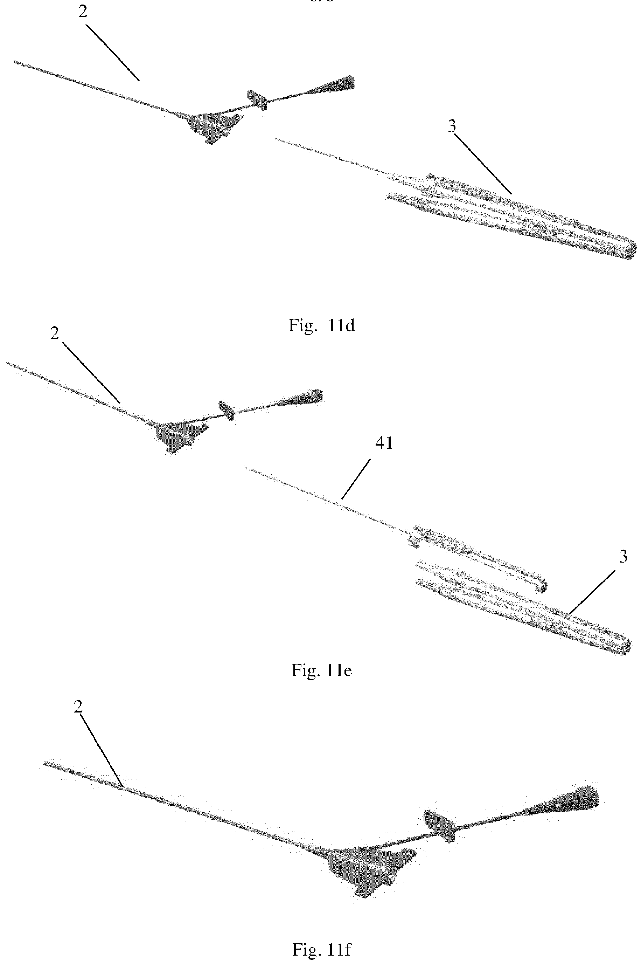

With reference to figures 11a to 11f it is shown a list of steps relating to the functioning of the device:
- insertion of the cannula-needle (5) in artery. The blood flows inside the valved catheter (fig. 11a) and is interrupted by the valve (27) inside the cylindrical seat (22) of the catheter assembly (2). Moreover, due to the high arterial pressure, the blood flows also in the extension of the catheter (24), where it can be interrupted due to the use of clamps (26) on the extension of the catheter assembly (2).
- Actuation of the Seldinger assembly (4) by a forward movement towards the needle end of the sliding actuation means (42) (fig. 11b). Consequently, the guidewire (41) is introduced in the artery of the patient through the cannula-needle (5).
- Push of the catheter assembly (2) inside the artery by a forward movement of the assembly itself, which can be obtained by exerting a force on the wings (23) of the assembly (figure 11c). Consequently, the catheter assembly (2) comes out from the outer sheath assembly (3) and the outer sheath assembly (3) begins to be divided in two parts (figure 11d).

- During removal of the outer sheath assembly (3) the Seldinger assembly (4), the cannula-needle (5) and the guidewire (41) are removed at the same time (figure 11e) by actuating the spring bulkhead (72) inside the convex seat (71).
- Use only of the catheter assembly (2) in the artery of the patient (figure 11f).

## Claims

1. Biomedical device (1) for carrying out an arterial catheterization comprising a catheter assembly (2), a Seldinger assembly (4), an outer sheath assembly (3) configured to contain thereinside both said catheter assembly (2) and said Seldinger assembly (4) when the device is assembled before its use, at least a cannula-needle (5) integral to the Seldinger assembly (4), valve means (27) to avoid blood backflow and safety means (7) for needle clamping (5), protection means (6) for the needle (5), said catheter assembly (2) comprising a thin tube (21), a nearly cylindrically shaped end (22) provided with a couple of side wings (23), an inner cavity obtained in said end (22), said device (1) being **characterized in that** said valve means (27) and said safety means (7) are located inside said outer sheath assembly (3) in the inner cavity obtained in said cylindrical end (22) of the catheter assembly (2).

2. Device (1) according to claim 1 **characterized in that** said outer sheath assembly (3) is provided with a couple of longitudinally developed side openings (31) configured to allow said couple of wings (23) to come out of the catheter assembly (2) .

3. Device (1) according to claims 1 or 2 **characterized in that** said outer sheath assembly (3) is provided with a longitudinally developed upper opening (32) through which it is possible to insert said Seldinger assembly (4).

4. Device (1) according to any one of claims from 1 to 3 **characterized in that** said Seldinger assembly (4) is made up of a sliding actuation means (32), to be introduced in the opening (32) of the outer sheath assembly (3), and of a guidewire (41) to be injected in the artery of the patient through the cannula-needle (5).

5. Device (1) according to any one of claims from 1 to 4 **characterized in that** said outer sheath assembly (3) is also configured to be divided in two parts, subjected to the actions of both the catheter assembly (2) and the Seldinger assembly (4) .

6. Device (1) according to any one of claims from 1 to 5, **characterized in that** said thin tube (21) of the catheter assembly (2) comes out from an open end of the outer sheath assembly (3).

7. Device (1) according to any one of claims from 1 to 6, **characterized in that** said catheter assembly (2) is also provided with a shunt tube (24) with a universal end (25), preferably of "luer lock" type and of at least one clamp (26).

8. Device (1) according to any one of claims from 1 to 7, **characterized in that** said clamping means (7) comprise a spring bulkhead (72) located inside a convex seat (71), said bulkhead being configured to be closed when it is crossed by the tip of the needle (5).

## Patentansprüche

1. Biomedizinische Vorrichtung (1) zur Durchführung einer arteriellen Katheterisierung, umfassend eine Katheter-Anordnung (2), eine Seldinger-Anordnung (4), eine äußere Hüllen-Anordnung (3), die so konfiguriert sind, dass sie sowohl die Katheter-Anordnung (2) als auch die Seldinger-Anordnung (4) enthalten, wenn die Vorrichtung vor ihrer Verwendung zusammengebaut wird, mindestens eine an der Seldinger-Anordnung (4) integral angeordnete Kanülennadel (5), Ventilmittel (27) zur Verhinderung des Blutrückflusses und Sicherheitsmittel (7) zum Festklemmen der Nadel (5), Schutzmittel (6) für die Nadel (5), wobei die Katheter-Anordnung (2) ein dünnes Rohr (21), ein nahezu zylindrisches Ende (22), das mit einem Paar seitlicher Flügel (23) versehen ist, und einen Innenraum umfasst, der im genannten Ende (22) vorhanden ist, wobei die Vorrichtung (1) **dadurch gekennzeichnet ist, dass** die Ventilmittel (27) und die Sicherheitsmittel (7) innerhalb der äußeren Hüllen-Anordnung (3) in dem inneren Hohlraum angeordnet sind, der im zylindrischen Ende (22) der Katheter-Anordnung (2) vorhanden ist.

2. Vorrichtung (1) nach Anspruch 1, **dadurch gekennzeichnet, dass** die äußere Hüllen-Anordnung (3) mit einem Paar seitlicher Öffnungen (31) versehen ist, die in Längsrichtung ausgebildet und so konfiguriert sind, um das Entfernen des Paares von Flügeln (23) von der Katheter-Anordnung (2) zu ermöglichen.

3. Vorrichtung (1) nach Anspruch 1 oder 2, **dadurch gekennzeichnet, dass** die äußere Hüllen-Anordnung (3) mit einer in Längsrichtung verlaufenden oberen Öffnung (32) versehen ist, durch die es möglich ist, die genannte Seldinger-Anordnung (4) einzuführen.

4. Vorrichtung (1) nach einem der Ansprüche 1 bis 3, **dadurch gekennzeichnet, dass** die Seldinger-Anordnung (4) aus einem verschiebbaren Betätigungsmittel (32) besteht, das in die Öffnung (32) der äußeren Hüllen-Anordnung (3) eingeführt wird und einen Führungsdraht (41) aufweist, der durch die Nadelkanüle (5) in die Arterie des Patienten injiziert wird.

5. Vorrichtung (1) nach einem der Ansprüche 1 bis 4, **dadurch gekennzeichnet, dass** die äußere Hüllen-Anordnung (3) außerdem so konfiguriert ist, dass sie in zwei Teile, abhängig von den Einflüssen sowohl der Katheter-Anordnung (2) als auch des Seldinger-Anordnung (4), geteilt werden kann.

6. Vorrichtung (1) nach einem der Ansprüche 1 bis 5, **dadurch gekennzeichnet, dass** das dünne Rohr (21) der Katheter-Anordnung (2) aus einem offenen Ende der äußeren Hüllen-Anordnung (3) austritt.

7. Vorrichtung (1) nach einem der Ansprüche 1 bis 6, **dadurch gekennzeichnet, dass** die Katheter-Anordnung (2) außerdem mit einem Shunt-Rohr (24) mit einem universalem Ende (25), vorzugsweise des "Luer-Lock"-Typs, versehen ist, und mindestens eine Klemme (26) aufweist.

8. Vorrichtung (1) nach einem der Ansprüche 1 bis 7, **dadurch gekennzeichnet, dass** die Klemmmittel (7) eine Federtrennwand (72) umfassen, die sich innerhalb eines konvexen Sitzes (71) befindet, wobei die Trennwand so konfiguriert ist, dass sie geschlossen werden kann, wenn sie von der Nadelspitze (5) gekreuzt wird.

## Revendications

1. Dispositif biomédical (1) pour réaliser un cathétérisme artériel comprenant un ensemble cathéter (2), un ensemble Seldinger (4), un ensemble gaine externe (3) configuré pour contenir à l'intérieur à la fois ledit ensemble cathéter (2) et ledit ensemble Seldinger (4) lorsque le dispositif est assemblé avant son utilisation, au moins une canule-aiguille (5) solidaire de l'ensemble Seldinger (4), des moyens de valve (27) pour éviter le reflux sanguin et des moyens de sécurité (7) de serrage de l'aiguille (5), des moyens de protection (6) pour l'aiguille (5), ledit ensemble cathéter (2) comprenant un tube mince (21), une extrémité de forme presque cylindrique (22) dotée d'une paire d'ailes latérales (23), une cavité intérieure obtenue dans ladite extrémité (22), ledit dispositif (1) étant **caractérisé en ce que** lesdits moyens de valve (27) et lesdits moyens de sécurité (7) sont situés à l'intérieur dudit ensemble gaine externe (3) dans la cavité interne obtenue dans ladite extrémité cylindrique (22) de l'ensemble cathéter (2) .

2. Dispositif (1) selon la revendication 1 **caractérisé en ce que** ledit ensemble gaine externe (3) est pourvu d'une paire d'ouvertures latérales (31) développées longitudinalement configurées pour permettre à la dite paire d'ailes (23) de sortir de l'ensemble cathéter. (2).

3. Dispositif (1) selon les revendications 1 ou 2 **caractérisé en ce que** ledit ensemble gaine externe (3) est pourvu d'une ouverture supérieure développée longitudinalement (32) à travers laquelle il est possible d'insérer ledit ensemble Seldinger (4).

4. Dispositif (1) selon l'une quelconque des revendications de 1 à 3 **caractérisé en ce que** ledit ensemble Seldinger (4) est constitué d'un moyen d'actionnement coulissant (32), à introduire dans l'ouverture (32) de l'ensemble gaine extérieure (3), et d'un fil guide (41) à injecter dans l'artère du patient à travers la canule-aiguille (5).

5. Dispositif (1) selon l'une quelconque des revendications 1 à 4 **caractérisé en ce que** ledit ensemble gaine externe (3) est également configuré pour être divisé en deux parties, soumises aux actions à la fois de l'ensemble cathéter (2) et de l'ensemble Seldinger (4).

6. Dispositif (1) selon l'une quelconque des revendications 1 à 5, **caractérisé en ce que** ledit tube mince (21) de l'ensemble cathéter (2) sort d'une extrémité ouverte de l'ensemble gaine externe (3).

7. Dispositif (1) selon l'une quelconque des revendications 1 à 6, **caractérisé en ce que** ledit ensemble cathéter (2) est également muni d'un tube shunt (24) à extrémité universelle (25), de préférence de type « luer lock » et d'au moins une pince (26).

8. Dispositif (1) selon l'une quelconque des revendications de 1 à 7, **caractérisé en ce que** lesdits moyens de serrage (7) comprennent une cloison à ressort (72) située à l'intérieur d'un siège convexe (71), ladite cloison étant configurée pour être fermée lorsque elle est traversée par la pointe de l'aiguille (5).
